Europäisches Patentamt

European Patent Office

Office européen des brevets

(11) Veröffentlichungsnummer: **0 328 978**

**A2**

(12) **EUROPÄISCHE PATENTANMELDUNG**

(21) Anmeldenummer: 89101998.6

(22) Anmeldetag: 06.02.89

(51) Int. Cl.⁴: **C07K 5/02 , C07D 239/22 , C07C 147/00 , C07C 149/23 , A61K 37/64 , A61K 31/505 , A61K 31/16**

Die Bezeichnung der Erfindung wurde geändert (Richtlinien für die Prüfung im EPA, A-III, 7.3).

(30) Priorität: 19.02.88 DE 3805156

(43) Veröffentlichungstag der Anmeldung: 23.08.89 Patentblatt 89/34

(84) Benannte Vertragsstaaten: AT BE CH DE ES FR GB IT LI NL SE

(71) Anmelder: **MERCK PATENT GESELLSCHAFT MIT BESCHRÄNKTER HAFTUNG Frankfurter Strasse 250 Postfach 4119 D-6100 Darmstadt(DE)**

(72) Erfinder: **Raddatz, Peter, Dr. Grafenstrasse 37 D-6100 Darmstadt(DE)** Erfinder: **Gante, Joachim, Dr. Stormstrasse 4 D-6100 Darmstadt 12(DE)** Erfinder: **Sombroek, Johannes, Dr. Robert-Koch-Strasse 32 D-6100 Darmstadt(DE)** Erfinder: **Schmitges, Claus J., Dr. Karolinger Strasse 5 D-6114 Gross-Umstadt(DE)** Erfinder: **Minck, Klaus-Otto, Dr. Büchestrasse 8 D-6105 Ober-Ramstadt(DE)**

(54) **Renin inhibierende Aminosäure-Derivate.**

(57) Neue Aminosäurederivate der Formel I

$R^1$-Z-$(CH_2)_m$-CO-$NR^2$-$CHR^3$-$CR^4$-$(CHR^5)_n$-CO-E-Q-Y     I

worin

$R^1$, Z, m, $R^2$, $R^3$, $R^4$, $R^5$, n, E, Q und Y die in Patentanspruch 1 angegebene Bedeutung haben, sowie ihre Salze hemmen die Aktivität des menschlichen Plasmarenins.

EP 0 328 978 A2

## Aminosäurederivate

Die Erfindung betrifft neue Aminosäurederivate der Formel I

$R^1$-Z-$(CH_2)_m$-CO-NR$^2$-CHR$^3$-CR$^4$-$(CHR^5)_n$-CO-E-Q-Y    I

worin

R$^1$    ($R^6$-$C_kH_{2k}$-S(O)$_x$-$C_rH_{2r}$)-L($R^7$-$C_pH_{2p}$)-CO- oder ($R^6$-$C_kH_{2k}$-S(O)$_x$-$C_rH_{2r}$)-L($R^7$-$C_pH_{2p}$)-CHOH-,

Z    -O-, -$CH_2$-, -CH=CH-, NR$^8$-, -NR$^8$-O-, -$CH_2$-O-, -$CH_2$-NR$^8$- oder -$CH_2$-S-,

E    0 bis 2 peptidartig miteinander verbundene Aminosäurereste ausgewählt aus der Gruppe bestehend aus Abu, Ala, Cal, His, Ile, Leu, Met, Nle, Nva, Phe, Trp, Tyr und Val,

Q    O oder NR$^9$

, Y    -$C_tH_{2t}$-R$^{1^*}$, -$C_tH_{2t}$-R$^{11}$ oder -$C_wH_{2w}$-(CR$^{12}$)$_s$-$C_tH_{2t}$-R$^{1^*}$,

R$^2$, R$^5$, R$^8$ und R$^9$    jeweils H oder A,

R$^3$, R$^6$, R$^7$ und R$^{1^*}$    jeweils H, A, Ar, Ar-Alkyl, Het, Hetalkyl, unsubstituiertes oder ein- oder mehrfach durch A, AO und/oder Hal substituier tes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R$^4$ und R$^{12}$    jeweils (H, OH), (H, NH$_2$) oder =O,

R$^{11}$    -SO$_3$H, -SO$_2$NH$_2$, -SO$_2$NHA, -SO$_2$NA$_2$, -NH$_2$, -NHA, -NA$_2$, -NA$_3^+$ An$^-$, -NH-C(=NH)-NH$_2$, -NH-C-(=NH)-NHCN, -NH-CO-NH$_2$, -NH-CO-NHA, -NH-CO-NA$_2$, -NH-CS-NH$_2$, -NH-CS-NHA oder -NH-CS-NA$_2$,

L    CH oder N,

k, p, r und t    jeweils 0, 1, 2, 3, 4 oder 5,

m    1, 2 oder 3,

n und s    jeweils 1 oder 2,

w    1, 2, 3, 4, 5, 6, 7, 8, 9 oder 10,

x    0, 1 oder 2,

Ar    unsubstituiertes oder ein- oder mehrfach durch A, OA, Hal, CF$_3$, OH, NO$_2$, Hydroxyalkyl, NH$_2$, NHA, NA$_2$, NHAc, SA, SO-A, SO$_2$-A, SO$_2$NH$_2$, SO$_2$NHA, CN, COOH, COOA, CONH$_2$, Aminoalkyl, HAN-alkyl, A$_2$N-alkyl, A$_3$N$^+$-alkyl An$^-$ und/oder Guanidinyl-alkyl substituiertes Phenyl oder unsubstituiertes Naphthyl,

Het    einen gesättigten oder ungesättigten 5-oder 6-gliedrigen heterocyclischen Rest mit 1-4 N-, O-und/oder S-Atomen, der mit einem Benzolring kondensiert und/oder ein-oder mehrfach durch A, OA, Hal, CF$_3$, OH, NO$_2$, Carbonylsauerstoff, NH$_2$, NHA, NA$_2$, NHAc, SA, SO-A, SO$_2$-A, COOH, COOA, CONH$_2$, CN, SO$_2$NH$_2$, NH-SO$_2$-A, Ar, Ar-alkyl, Ar-alkenyl, Hydroxyalkyl und/oder Aminoalkyl substituiert sein kann und/oder dessen N-und/oder S-Heteroatome auch oxydiert sein können,

Hal    F, Cl, Br oder J,

Ac    A-CO-, Ar-CO- oder A-NH-CO-,

An$^-$    ein Anion,

A    Alkyl mit 1-8 C-Atomen und

-alkyl-    eine Alkylengruppe mit 1-8 C-Atomen bedeuten,

worin ferner an Stelle einer oder mehrerer -NH-CO-Gruppen auch eine oder mehrere -NA-CO-Gruppen stehen können,

sowie deren Salze.

Ähnliche Verbindungen sind aus der DE-A-3619508 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B Pepsin und Kathepsin D) sind in der Regel wesentlich höhere Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor- und nachstehend aufgeführten Abkürzungen von Aminosäureresten stehen für die Reste -NR´-

2

R''-CO-, in der Regel -NH-CHR-CO- (worin R, R' und R'' die für jede Aminosäure bekannte spezifische Bedeutung haben) folgender Aminosäuren:

| Abu | 2-Aminobuttersäure |
|---|---|
| AHCH | 4S-Amino-3S-hydroxy-6-cyclohexyl-hexansäure |
| AHCP | 4S-Amino-3S-hydroxy-5-cyclohexyl-pentansäure |
| AHPP | 4S-Amino-3S-hydroxy-5-phenyl-pentansäure |
| Ala | Alanin |
| ßAla | ß-Alanin |
| Cal | 3-Cyclohexylalanin |
| DACH | 3S,4S-Diamino-6-cyclohexyl-hexansäure |
| DACP | 3S,4S-Diamino-5-cyclohexyl-pentansäure |
| DAMH | 3S,4S-Diamino-6-methyl-heptansäure |
| DAPP | 3S,4S-Diamino-5-phenyl-pentansäure |
| Gly | Glycin |
| His | Histidin |
| Ile | Isoleucin |
| Leu | Leucin |
| Met | Methionin |
| Nle | Norleucin |
| Nva | Norvalin |
| Phe | Phenylalanin |
| Sta | Statin |
| Trp | Tryptophan |
| Tyr | Tyrosin |
| Val | Valin. |

Ferner bedeutet nachstehend:

| AMPA | N-4-Amino-2-methyl-5-pyrimidinylmethyl-amid |
|---|---|
| BOC | tert.-Butoxycarbonyl |
| BOM | Benzyloxymethyl |
| CBZ | Benzyloxycarbonyl |
| DMF | Dimethylformamid |
| DNP | 2,4-Dinitrophenyl |
| imi-DNP | 2,4-Dinitrophenyl in 1-Stellung des Imidazolrings |
| ETOC | Ethoxycarbonyl |
| FMOC | 9-Fluorenylmethoxycarbonyl |
| POA | Phenoxyacetyl |
| DCCI | Dicyclohexylcarbodiimid |
| HOBt | 1-Hydroxybenzotriazol. |

Sofern die vorstehend genannten Aminosäuren in mehreren enantiomeren Formen auftreten können, so sind vor- und nachstehend, z.B als Bestandteil der Verbindungen der Formel I, alle diese Formen und auch ihre Gemische (z.B die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$R^1\text{-}G^1\text{-}OH$

worin $G^1$

(a) fehlt und gleichzeitig $R^1$ $(R^6\text{-}C_kH_{2k}\text{-}S(O)_x\text{-}C_rH_{2r})\text{-}L(R^7\text{-}C_pH_{2p})\text{-}CO\text{-}$bedeutet,

(b) $\text{-}Z\text{-}(CH_2)_m\text{-}CO\text{-}$,

3

(c) -Z-(CH$_2$)$_m$-CO-W-,

(d) -Z-(CH$_2$)$_m$-CO-W-E$^1$-,

(e) -Z-(CH$_2$)$_m$-CO-W-E- und

W    -NR$^2$-CHR$^3$-CR$^4$-(CHR$^5$)$_n$-CO- bedeuten

oder eines ihrer reaktionsfähigen Derivate

mit einer Verbindung der Formel III

H-G$^2$     III

worin

G$^2$

(a) -Z$^1$-(CH$_2$)$_m$-CO-W-E-Q-Y

(b) -W-E-Q-Y,

(c) -E-Q-Y,

(d) -E$^2$-Q-Y,

(e) -Q-Y,

Z$^1$    -O-, -NR$^8$-oder -NR$^8$-O- und

E$^1$ + E$^2$    zusammen E bedeuten

oder einem ihrer reaktionsfähigen Derivate umsetzt,

und/oder daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Amino- oder Hydroxygruppe acyliert und/oder eine Ketogruppe zu einer CHOH-Gruppe reduziert oder zu einer CH(NH$_2$)-Gruppe reduktiv aminiert und/oder eine Verbindung der Formel I durch Behandeln mit einer Säure in eines ihrer Salze überführt.

Vor- und nachstehend haben die Reste bzw. Parameter R$^1$ bis R$^{12}$, Z, E, Q, Y, L, k, m, n, p, r, s, t, w, x, Ar, Het, Hal, Ac, An, A, G$^1$, G$^2$, Z$^1$, E$^1$, E$^2$ und W die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin, Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethyl butyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B 1-, 2- oder 3-Methylcyclopentyl, 1-, 2-, 3- oder 4-Methylcyclohexyl.

Dementsprechend bedeutet Cycloalkylalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl o-, m- oder p-Jodphenyl, o-, m- oder p-Trifluormethylphenyl, o-, m- oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m- oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, o-, m- oder p-Dimethylaminomethylbenzyl, o-, m- oder p-Guanidinomethylbenzyl, 1- oder 2-Naphthylmethyl.

Het ist vorzugsweise 2- oder 3-Furyl, 2- oder 3-Thienyl, 1-, 2- oder 3-Pyrryl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder

4

5-Isothiazolyl, 2-, 3- oder 4-Pyridyl, 2-, 4-, 5- oder 6-Pyrimidyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6-oder 7-Benzisoxazolyl, 2-, 4-, 5-, 6- oder 7-Benzthiazolyl, 2-, 4-, 5-, 6- oder 7-Benzisothiazolyl, 4-, 5-, 6-oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5-oder -6-pyridyl, 1,2,3,6-Tetrahydro-1-, -2-, -3-, -4-, -5-oder -6-pyridyl, 1-, 2-, 3- oder 4-Piperidinyl, 2-, 3-oder 4-Morpholinyl, Tetrahydro-2-, -3- oder -4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-methyl-4-pyrimidinyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl, 2-Oxo-pyrrolidino, 2-Oxo-piperidino, 2,5-Dioxopyrrolidino, 3-Benzyl-2,5-dioxopyrrolidino. L ist vorzugsweise CH. Die Parameter k, m, p und r sind vorzugsweise jeweils 0, 1 oder 2; n und s sind vorzugsweise 1; x ist vorzugsweise 2. Die Gruppen $C_tH_{2t}$ und $C_wH_{2w}$ sind vorzugsweise jeweils -$CH_2$-, -($CH_2$)$_2$-, -$CH(CH_3)$-, -CH (Isobutyl) oder CH (sek.-Butyl); die Gruppe $C_tH_{2t}$ kann bevorzugt auch fehlen.

$R^1$ bedeutet vorzugsweise ($R^6$-$C_kH_{2k}$ -S(O)$_x$-$C_rH_{2r}$)-L($R^7$-$C_pH_{2p}$)-CO-, insbesondere ($R^6$-$C_kH_{2k}$-SO$_2$-$C_rH_{2r}$)-L($R^7$-$C_pH_{2p}$)-CO-, im einzelnen bevorzugt ($R^6$-$C_kH_{2k}$-SO$_2$-$C_rH_{2r}$)-CH($R^7$-$C_pH_{2p}$)-CO- wie (A-SO$_2$-$C_rH_{2r}$)-CH($R^7$-$C_pH_{2p}$)-CO- und speziell (A-SO$_2$-$CH_2$)-CH($R^7$-$CH_2$)-CO-, worin A bevorzugt 1-6 C-Atome enthält und $R^7$ bevorzugt A, Ar oder Cycloalkyl bedeutet, z. B. 2-Benzyl-3-tert.-butylsulfonyl-propionyl, 2-p-Methoxybenzyl-3-tert.-butylsulfonyl-propionyl, 2-(1- oder 2-Naphthyl-methyl)-3-tert.-butylsulfonyl-propionyl, 2-(tert.-Butylsulfonyl-methyl)-propionyl, -butyryl, -isobutyryl, -valeryl, -3-methylbutyryl, -hexanoyl, -4-methyl-pentanoyl, -heptanoyl, -4,4-dimethylpentanoyl, -octanoyl, -nonanoyl, -decanoyl. $R^1$ kann z. B. aber auch bedeuten 1-Hydroxy-2-benzyl-3-tert.-butylsulfonylpropyl, 2-Benzyl-3-tert.-butylthio-propionyl, 2-Benzyl-3-tert.-butylsulfinyl-propionyl.

Z bedeutet vorzugsweise -$NR^8$-, insbesondere -NH- oder -N(CH$_3$)-, ferner bevorzugt -$CH_2$-; -$CH_2$-O-; -$CH_2$-$NR^8$-, insbesondere -$CH_2$-NH-; oder -$CH_2$-S-. Dementsprechend bedeutet die Gruppe -Z-($CH_2$)$_m$-CO- vorzugsweise Gly oder ßAla, ferner bevorzugt -$CH_2CH_2$CO-, -$CH_2$-O-$CH_2$-CO-, -$CH_2$-NH-$CH_2$-CO- ( = -$CH_2$-Gly-), -NH-CO-, -NH-($CH_2$)$_3$-CO-, -$CH_2$-NH-CO-, -NH-O-$CH_2$CO- oder -$CH_2$-S-$CH_2$-CO-.

$R^2$ und $R^5$ bedeuten vorzugsweise H oder Methyl, ferner Ethyl, Propyl, Isopropyl, Butyl oder Isobutyl. Falls n = 2 ist, können die beiden Reste $R^5$ gleich oder von einander verschieden sein; vorzugsweise ist in diesem Fall der eine Rest $R^5$ H, der andere A, insbesondere Isopropyl, die Gruppe -($CHR^5$)$_n$- also bevorzugt -$CH_2$-CH(Isopropyl)-.

$R^3$ bedeutet vorzugsweise Cyclohexylmethyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Phenyl, Benzyl, p-Chlorbenzyl, 2-Cyclohexylethyl, Bicyclo[2,2,1]heptyl-2-methyl oder 6,6-Dimethylbicyclo[3,1,1]heptyl-2-methyl.

$R^4$ ist vorzugsweise (H, OH).

Die Gruppe W bedeutet vorzugsweise -NH-CHR³-CHOH-CH₂-CO-, insbesondere AHCP, AHCH, Sta oder AHPP. Die Gruppe W bedeutet ferner bevorzugt -NH-CHR³-CH(NH₂)-CH₂-CO-, insbesondere DACP, DACH, DAMH oder DAPP.

Die Gruppe W besitzt mindestens ein chirales Zentrum. In den Gruppen $R^1$ bis $R^5$, Z, E, Q und Y können weitere chirale Zentren vorhanden sein. Die Verbindungen der Formel I können daher in verschiedenen - optisch-inaktiven oder optisch-aktiven - Formen vorkommen. Die Formel I umschließt alle diese Formen. Falls W -NH-CHR³-CR⁴-CH₂-CO- mit $R^4$ = (H, OH) oder (H, NH₂) bedeutet, sind die 3S-Hydroxy-4S-amino-Enantiomeren bzw. 3S,4S-Diamino-Enantiomeren bevorzugt. Die Abkürzungen AHCP, AHCH, Sta, AHPP, DACP, DACH, DAMH und DAPP beziehen sich immer auf die 3S,4S-Formen.

Die oben erwähnten Cycloalkyl- und Phenylgruppen tragen vorzugsweise 1 bis 3, insbesondere 1 oder 2 Substituenten. E bedeutet vorzugsweise einen der genannten Aminosäurereste, insbesondere Ile oder Leu; ferner ist E bevorzugt abwesend oder bedeutet bevorzugt Abu, Cal, Met, Nle, Nva, Phe oder Val.

Q ist vorzugsweise NR⁹, insbesondere NH oder N(CH₃).

Y ist vorzugsweise $-C_tH_{2t}-R^{1^\circ}$ oder $C_tH_{2t}-R^{11}$ , insbesondere $-CH_2R^{1^\circ}$ , $-CH_2R^{11}$ oder $-CH_2CH_2R^{11}$. Dabei bedeutet $R^{1^\circ}$ vorzugsweise H, A, Ar oder Het, im einzelnen bevorzugt H, Alkyl mit 3-5 C-Atomen, Phenyl, o-, m- oder p-Aminomethylphenyl, o-, m- oder p-Guanidinomethylphenyl, o-, m-oder p-Dialkylaminomethylphenyl wie o-, m- oder p-Dimethylaminomethylphenyl, 2-, 3- oder 4-Pyridyl, 2-Hydroxy-4,6-dimethyl-3-pyridyl, 4-Amino-2-methyl-5-pyrimidinyl oder 2-Amino-5,6-dimethyl-3-pyrazinyl. $R^{11}$ bedeutet vorzugsweise -SO₃H, -SO₂NH₂, -NA₂, -NA₃⁺ An⁻, -NH-C(=NH)-NH₂, -NH-CO-NHA oder -NH-CS-NHA, wobei A bevorzugt CH₃ ist.

Einige besonders bevorzugte Bedeutungen der Gruppe Q-Y sind -NH-CH₂-(4-amino-2-methyl-5-pyrimidinyl), -NH-CH₂-(2-amino-5,6-dimethyl-3-pyrazinyl) und -NH-CH₂-(3-pyridyl), ferner -NH-A.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ii ausgedrückt werden, die der Formel I entsprechen, worin jedoch

in Ia

$R^1$    R⁵-SO₂-C_rH₂_r-CH(CH₂R⁷)-CO- bedeutet;

in Ib

$R^1$    A-SO₂-CH₂-CH(CH₂R⁷)-CO-,
$R^7$    A, Ar oder Cyclohexyl und
Ar    Phenyl, Methoxyphenyl oder Naphthyl bedeuten;

in Ic

$R^1$    A-SO₂-CH₂-CH(CH₂Ar)-CO- und
Ar    Phenyl oder Naphthyl bedeuten;

in Id

Z    -NH- bedeutet;

in Ie

-Z-(CH₂)_m-CO-    Gly oder βAla bedeutet;

in If

-NR²-CHR³-CR⁴-(CHR⁵)_n-CO- (= W) AHCP bedeutet;

in Ig

E    fehlt oder Ile oder Leu bedeutet;

in Ih

R¹    A-SO₂-CH₂-CH(CH₂-R⁷)-CO-,
R⁷    A, Ar oder Cyclohexyl,
Ar    Phenyl, Methoxyphenyl oder Naphthyl,
W    AHCP, -NH-CH(isobutyl)-CHOH-CH₂CH(isopropyl)-CO- oder -NH-CH(CH₂-cyclohexyl)CHOH-CH₂-CH-(isopropyl)-CO- bedeuten und E    fehlt oder Ile oder Leu bedeutet;

in Ii

R¹    2-Benzyl-3-tert.-butylsulfonyl-propionyl,
-Z-(CH₂)ₘ-CO-    Gly oder βAla,
W    AHCP oder -NH-CH(CH₂-cyclohexyl)-CHOH-CH₂-CH(isopropyl)-CO- bedeuten und
E    fehlt oder Ile bedeutet.
Insbesondere sind bevorzugt Verbindungen der Teilformeln:
I* sowie Ia* bis Ii*, die den Formeln I sowie Ia bis Ii entsprechen, worin jedoch
Q    NH,
Y    A oder -CH₂R¹°
R¹°    o-, m- oder p-Aminomethylbenzyl, o-, m- oder p-Guanidinomethylbenzyl, 3-Pyridyl, 4-Amino-2-methyl-5-pyrimidinyl oder 2-Amino-5,6-dimethyl-3-pyrazinyl bedeuten.
I' sowie Ia' bis Ii', die den Formeln I sowie Ia bis Ii entsprechen, worin jedoch
Q    NH,
Y    A oder 4-Amino-2-methyl-5-pyrimidinylmethyl bedeuten.
Die Verbindungen der Formel I und auch die Ausgangsstoffe zu ihrer Herstellung werden im übrigen nach an sich bekannten Methoden hergestellt, wie sie in der Literatur (z.B in den Standardwerken wie Houben-Weyl, Methoden der organischen Chemie, Georg-Thieme-Verlag, Stuttgart; ferner DE-A-3619508, EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B solche die der Formel I entsprechen, aber an Stelle einer His-Gruppe eine N(im)-R'-His-Gruppe (worin R' eine Aminoschutzgruppe bedeutet, z.B BOM oder DNP) enthalten, oder solche der Formel R¹-Z-(CH₂)ₘ-CO-NR²-CHR³-CH(NHR')-(CHR⁵)ₙ-CO-E-Q-Y.

Ferner sind Ausgangsstoffe bevorzugt, die an Stelle des H-Atoms einer Hydroxygruppe eine Hydroxyschutzgruppe tragen, z.B solche der Formel R¹-Z-(CH₂)ₘ-CO-NR²-CHR³-CHOR"-(CHR⁵)ₙ-CO-E-Q-Y, worin R" eine Hydroxyschutzgruppe bedeutet.

Es können auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B DNP), Aralkoxymethyl- (z.B BOM) oder Aralkylgruppen (z.B Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammen hang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B in den genannten Standardwerken und Patentanmeldungen beschrieben sind, z.B auch nach der Festphasenmethode nach Merrifield.

Das In-Freiheit-Setzen der Verbindungen der Formel I aus ihren funktionellen Derivaten gelingt - je nach der benutzten Schutzgruppe - z.B mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol-oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B bevorzugt mit 40%iger Trifluoressigsäure in Dichlormethan oder mit etwa 3 bis 5 n HCl in Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B auch mit einer etwa 3-10%igen Lösung von 2-Mercaptoethanol in DMF/Wasser bei 15-30°.

Hydrogenolytisch entfernbare Schutzgruppen. (z.B BOM, CBZ oder Benzyl) können z.B durch Behandeln mit Wasserstoff in Gegenwart eines Katalysators (z.B eines Edelmetall katalysators wie Palladium, zweckmäßig auf einem Träger wie Kohle) abgespalten werden. Als Lösungsmittel eignen sich dabei die oben angegebenen, insbesondere z.B Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B gut an 5-10%igem Pd-C in Methanol bei 20-30°.

Verbindungen der Formel I können auch durch direkte Peptidsynthese aus einer Carbonsäure- (Formel II) und einer Aminkomponente (Formel III) erhalten werden. Als Carbonsäurekomponenten eignen sich z. B. solche der Teilformeln $(R^6-C_kH_{2k}-S(O)_x-C_rH_{2r})-L(R^7-C_pH_{2p})-COOH$, $R^1-Z-(CH_2)_m-COOH$, $R^1-Z-(CH_2)_m-CO-W-OH$ oder $R^1-Z-(CH_2)_m-CO-W-E-OH$, als Aminkomponenten solche der Teilformeln $H-Z^1-(CH_2)_m-CO-W-E-Q-Y$, $H-W-E-Q-Y$, $H-E-Q-Y$ oder $H-Q-Y$. Die Peptidbindung kann aber auch innerhalb der Gruppe E geknüpft werden; dabei wird eine Carbonsäure der Formel $R^1-Z-(CH_2)_m-CO-W-E^1-OH$ mit einer Aminoverbindung der Formel $H-E^2-Q-Y$ umgesetzt, wobei $E^1 + E^2 = E$ ist. Dabei arbeitet man zweckmäßig nach üblichen Methoden der Peptid-Synthese, wie sie z.B in Houben-Weyl, l.c., Band 15/II, Seiten 1-806 (1974) beschrieben sind.

Die Reaktion gelingt vorzugsweise in Gegenwart eines Dehydratisierungsmittels, z.B eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden. z.B. solche, in denen reaktive Gruppen intermediär durch Schutzgruppen blockiert sind. Die Aminosäurederivate III können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine AcNH- oder eine AOOC-Gruppe enthält, in die entsprechende Verbindung der Formel I umgewandelt werden, die stattdessen eine $H_2N$- oder eine HOOC-Gruppe enthält, zweckmäßig durch selektive Solvolyse nach einer der oben angegebenen Methoden.

Umgekehrt kann man nach üblichen Methoden eine freie Amino- oder Hydroxygruppe acylieren, z. B. nach einer der oben für die Umsetzung von II mit III angegebenen Methoden.

Weiterhin können z.B Ketoverbindungen der Formel I ($R^4$ = O) zu Verbindungen der Formel I ($R^4$ = (H, OH)) reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°.

Ketoverbindungen der Formel I ($R^4$ = O) können auch durch reduktive Aminierung in Verbindungen der Formel I ($R^4$ = H, $NH_2$) übergeführt werden. Man kann ein- oder mehrstufig reduktiv aminieren. So kann man z.B die Ketoverbindung mit Ammoniumsalzen, z.B Ammoniumacetat, und $NaCNBH_3$ behandeln, vorzugsweise in einem inerten Lösungsmittel, z.B einem Alkohol wie Methanol, bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B durch katalytische Hydrierung an Raney-Nickel, zum Amin zu reduzieren.

Eine Base der Formel I kann mit einer Säure in das zugehörige Säureadditionssalz übergeführt wrden. Für diese Umsetzung kommen insbesondere Säuren in Frage, die physiologisch unbedenkliche Salze liefern, So können anorganische Säuren verwendet werden, z.B Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, PolyethylenGlykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B Fluorchlorkohlen wasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B Ethanol. Inhalationslösungen können mit Hilfe üblicher Inhalatoren verabfolgt werden. Die neuen Verbindungen können auch lyophilisiert und die erhaltenen Lyophilisate z.B zur Herstllung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B ein oder mehrere Vitamine.

Die erfindungsgemäßen Substanzen werden in der Regel in Analogie zu anderen bekannten, im Handel befindlichen Peptiden, insbesondere aber in Analogie zu den in der DE-A-3619508 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg, insbesondere 10 und 100 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt. Vor- und nachstehend sind alle Temperaturen in $^\circ$C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, filtriert, dampft ein und reinigt durch Chromatographie an Kieselgel und/oder Kristallisation.

Beispiel 1

Ein Gemisch von 953 mg 5-[N-3S-Hydroxy-4S-[(2-benzyl-3-tert.-butylsulfonyl-propionyl)-Glycyl-amino]-5-cyclohexyl-pentanoyl-amino-L-isoleucyl-N-(imi)-2,4-dinitrophenyl-L-histidin-amid["(2-Benzyl-3-tert.-butylsulfonyl-propionyl)-Gly-AHCP-Ile-(imi-DNP-His)-NH$_2$"; erhältlich durch Kondensation von BOC-Ile-OH mit H-(imi-DNP-His)-NH$_2$ zu BOC-Ile-(imi-DNP-His)-NH$_2$, Abspaltung der BOC-Gruppe, Kondensation mit BOC-AHCP-OH zu BOC-AHCP-Ile-(imi-DNP-His-NH$_2$), erneute Abspaltung der BOC-Gruppe, Kondensation mit BOC-Gly-OH zu BOC-Gly-AHCP-Ile-(imi-DNP-His)-NH$_2$, erneute Abspaltung der BOC-Gruppe und Kondensation mit 2-Benzyl-3-tert.-butylsulfonyl-propionsäure], 860 mg 2-Mercaptoethanol, 10 ml DMF und 10 ml Wasser wird unter Rühren bei 20$^\circ$ mit wässeriger Na$_2$CO$_3$-Lösung auf pH 8 eingestellt und 3 Std. bei 20$^\circ$ gerührt. Nach üblicher Aufarbeitung erhält man (2-Benzyl-3-tert.-butyl-propionyl)Gly-AHCP-Ile-His-NH$_2$.

Beispiel 2

Man löst 1 g 3S-CBZ-amino-4S-(2-benzyl-3-tert.-butylsulfonyl-propionyl-Gly-amino)-5-cyclohexyl-pentanoyl-Ile-AMPA in 10 ml Ethanol, hydriert an 0,5 g 10%igem Pd-C bei 20$^\circ$ und 1 bar bis zum Stillstand der H$_2$-Aufnahme, filtriert, dampft ein und erhält nach chromatographischer Reinigung (2-Benzyl-3-tert.-butylsulfonyl-propionyl)-Gly-DACP-Ile-AMPA.

Analog erhält man durch Hydrogenolyse der entsprechenden 3S-CBZ-amino-derivate:
(2-Benzyl-3-tert.-butylsulfonyl-propionyl)-Gly-DACP-Leu-AMPA
(2-Benzyl-3-tert.-butylsulfonyl-propionyl)-βAla-DACP-Ile-AMPA
(2-Benzyl-3-tert.-butylsulfonyl-propionyl)-βAla-DACP-Leu-AMPA
(2-Benzyl-3-tert.-butylsulfonyl-propionyl)-Gly-DACH-Ile-AMPA
(2-Benzyl-3-tert.-butylsulfonyl-propionyl)-Gly-DACH-Leu-AMPA
(2-Benzyl-3-tert.-butylsulfonyl-propionyl)-βAla-DACH-Ile-AMPA
(2-Benzyl-3-tert.-butylsulfonyl-propionyl)-βAla-DACH-Leu-AMPA
(2-Benzyl-3-tert.-butylsulfonyl-propionyl)-Gly-DAMH-Ile-AMPA
(2-Benzyl-3-tert.-butylsulfonyl-propionyl)-Gly-DAMH-Leu-AMPA
(2-Benzyl-3-tert.-butylsulfonyl-propionyl)-βAla-DAMH-Ile-AMPA
(2-Benzyl-3-tert.-butylsulfonyl-propionyl)-βAla-DAMH-Leu-AMPA
(2-Benzyl-3-tert.-butylsulfonyl-propionyl)-Gly-DAPP-Ile-AMPA
(2-Benzyl-3-tert.-butylsulfonyl-propionyl)-Gly-DAPP-Leu-AMPA
(2-Benzyl-3-tert.-butylsulfonyl-propionyl)-βAla-DAPP-Ile-AMPA
(2-Benzyl-3-tert.-butylsulfonyl-propionyl)-βAla-DAPP-Leu-AMPA.

Beispiel 3

Eine Lösung von 5,05 g H-Gly-AHCP-Ile-AMPA [erhältlich durch Kondensation von BOC-Gly-AHCP-OH mit H-Ile-AMPA und anschließende Abspaltung der BOC-Gruppe] in 60 ml Dichlormethan wird unter Rühren mit 2,84 g 2-Benzyl-3-tert.-butylsulfonyl-propionsäure, 1,35 g HOBt und einer Lösung von 2,06 g DCCI in 50 ml Dichlormethan versetzt. Man rührt 18 Std. bei 2-7°, filtriert und dampft das Filtrat ein. Nach üblicher Aufarbeitung erhält man (2-Benzyl-3-tert.-butylsulfonyl-propionyl)-Gly-AHCP-Ile-AMPA ("C"), F. 139-140°. Hydrochlorid, F. 134-135°.

Analog erhält man

(2-Benzyl-3-tert.-butylsulfonyl-propionyl)-Gly-AHCP-Leu-AMPA
[2-(1-Naphthylmethyl)-3-tert.-butylsulfonyl-propionyl]-Gly-AHCP-Ile-AMPA
[2-(1-Naphthylmethyl)-3-tert.-butylsulfonyl-propionyl]-Gly-AHCP-Leu-AMPA
[2-(1-Naphthylmethyl)-3-tert.-butylsulfonyl-propionyl]-ßAla-AHCP-Ile-AMPA
[2-(1-Naphthylmethyl)-3-tert.-butylsulfonyl-propionyl]-ßAla-AHCP-Leu-AMPA ·
[2-(tert.Butylsulfonylmethyl)-heptanoyl]-Gly-AHCP-Ile-AMPA
|2-(tert.Butylsulfonylmethyl)-heptanoyl]-Gly-AHCP-Leu-AMPA
[2-(tert.Butylsulfonylmethyl)-heptanoyl]-ßAla-AHCP-Ile-AMPA
[2-(tert.Butylsulfonylmethyl)-heptanoyl]-ßAla-AHCP-Leu-AMPA
(2-p-Methoxybenzyl-3-tert.-butylsulfonyl-propionyl)-Gly-AHCP-Ile-AMPA
(2-p-Methoxybenzyl-3-tert.-butylsulfonyl-propionyl)-Gly-AHCP-Leu-AMPA
(2-p-Methoxybenzyl-3-tert.-butylsulfonyl-propionyl)-ßAla-AHCP-Ile-AMPA
(2-p-Methoxybenzyl-3-tert.-butylsulfonyl-propionyl)-ßAla-AHCP-Leu-AMPA
(2-Cyclohexylmethyl-3-tert.-butylsulfonyl-propionyl)-Gly-AHCP-Ile-AMPA
(2-Cyclohexylmethyl-3-tert.-butylsulfonyl-propionyl)-Gly-AHCP-Leu-AMPA
(2-Cyclohexylmethyl-3-tert.-butylsulfonyl-propionyl)-ßAla-AHCP-Ile-AMPA
(2-Cyclohexylmethyl-3-tert.-butylsulfonyl-propionyl)-ßAla-AHCP-Leu-AMPA
2-Isopropylsulfinylmethyl-hexanoyl-Gly-AHCP-Phe-OMe 2-(2-Naphthylmethyl)-3-(2-cyclopentylethyl-thio-methyl)-propionyl-Gly-AHCP-OEt
2-Cyclopropylmethyl-3-benzylsulfonylmethyl-propionyl-Gly-AHCP-(p-acetamidobenzylester).

Beispiel 4

Analog Beispiel 3 erhält man aus 2-Benzyl-3-tert.-butylsulfonyl-propionyl-ßAla-OH und H-AHCP-Ile-AMPA das (2-Benzyl-3-tert.-butylsulfonyl-propionyl)-ßAla-AHCP-Ile-AMPA, F. 148-149°. Methansulfonat, F. 116-117°.

Analog erhält man

2-Isopropyl-4S-hydroxy -5S-[(2-benzyl-3-tert.-butylsulfonyl-propionyl)-Gly-amino]-6-cyclohexyl-hexansäure-N-butylamid, 2 Isomere "A", F. 136°, und "B", F. 96°;
2-Isopropyl-4S-hydroxy-5S-[(2-benzyl-3-tert.-butylsulfonyl-propionyl)-Gly-amino]-6-cyclohexyl-hexanoyl-Ile-AMPA
2-Isopropyl-4S-hydroxy-5S-[(2-benzyl-3-tert.-butylsulfonyl-propionyl)-Gly-amino]-6-cyclohexyl-hexanoyl-Leu-AMPA
2-Isopropyl-4S-hydroxy-5S-[(2-benzyl-3-tert.-butylsulfonyl-propionyl)-Gly-amino]-6-cyclohexyl-hexanoyl-Ile-(N-2-amino-5,6-dimethyl-3-pyrazinyl-methyl-amid)
2-Isopropyl-4S-hydroxy-5S-[(2-benzyl-3-tert.-butylsulfonyl-propionyl)-Gly-amino]-6-cyclohexyl-hexanoyl-Leu-(N-2-amino-5,6-dimethyl-3-pyrazinyl-methyl-amid)
2-Isopropyl-4S-hydroxy-5S-[(2-benzyl-3-tert.-butylsulfonyl-propionyl)-Gly-amino]-6-cyclohexyl-hexanoyl-Ile-(N-3-aminomethyl-benzyl-amid)
2-Isopropyl-4S-hydroxy-5S-[(2-benzyl-3-tert.-butylsulfonyl-propionyl)-Gly-amino]-6-cyclohexyl-hexanoyl-Leu-(N-3-aminomethyl-benzyl-amid)
2-Isopropyl-4S-hydroxy-5S-[(2-benzyl-3-tert.-butylsulfonyl-propionyl)-Gly-amino]-6-cyclohexyl-hexanoyl-Ile-(N-3-guanidinomethyl-benzyl-amid)
2-Isopropyl-4S-hydroxy-5S-[(2-benzyl-3-tert.-butylsulfonyl-propionyl)-Gly-amino]-6-cyclohexyl-hexanoyl-Leu-(N-3-guanidinomethyl-benzyl-amid)
2-Isopropyl-4S-hydroxy-5S-[(2-benzyl-3-tert.-butylsulfonyl-propionyl)-ßAla-amino]-6-cyclohexyl-hexanoyl-Ile-AMPA
2-Isopropyl-4S-hydroxy-5S-[(2-benzyl-3-tert.-butylsulfonyl-propionyl)-ßAla-amino]-6-cyclohexyl-hexanoyl-

11

Leu-AMPA

2-Isopropyl-4S-hydroxy-5S-[(2-benzyl-3-tert.-butylsulfonyl-propionyl)-ßAla-amino]-6-cyclohexyl-hexanoyl-Ile-
(N-2-amino-5,6-dimethyl-3-pyrazinyl-methyl-amid)

2-Isopropyl-4S-hydroxy-5S-[(2-benzyl-3-tert.-butylsulfonyl-propionyl)-ßAla-amino]-6-cyclohexyl-hexanoyl-
Leu-(N-2-amino-5,6-dimethyl-3-pyrazinyl-methyl-amid.)

2-Isopropyl-4S-hydroxy-5S-[(2-benzyl-3-tert.-butylsulfonyl-propionyl)-ßAla-amino]-6-cyclohexyl-hexanoyl-Ile-
(N-3-aminomethyl-benzyl-amid)

2-Isopropyl-4S-hydroxy-5S-[(2-benzyl-3-tert.-butylsulfonyl-propionyl)-ßAla-amino]-6-cyclohexyl-hexanoyl-
Leu-(N-3-aminomethyl-benzyl-amid)

2-Isopropyl-4S-hydroxy-5S-[(2-benzyl-3-tert.-butylsulfonyl-propionyl)-Gly-amino]-6-cyclohexyl-hexanoyl-Ile-
(N-3-guanidinomethyl-benzyl-amid)

2-Isopropyl-4S-hydroxy-5S-[(2-benzyl-3-tert.-butylsulfonyl-propionyl)-Gly-amino]-6-cyclohexyl-hexanoyl-Leu-
(N-3-guanidinomethyl-benzyl-amid)

(2-Benzyl-3-tert.-butylsulfonyl-propionyl)-Gly-AHCP-Leu-(N-pentyl-amid)

(2-Benzyl-3-tert.-butylsulfonyl-propionyl)-Gly-AHCP-Ile-(N-2-amino-5,6-dimethyl-3-pyrazinyl-methyl-amid)

(2-Benzyl-3-tert.-butylsulfonyl-propionyl)-Gly-AHCP-Leu-(N-2-amino-5,6-dimethyl-3-pyrazinyl-methyl-amid)

(2-Benzyl-3-tert.-butylsulfonyl-propionyl)-Gly-AHCP-Ile-(N-3-aminomethyl-benzyl-amid)

(2-Benzyl-3-tert.-butylsulfonyl-propionyl)-Gly-AHCP-Leu-(N-3-aminomethyl-benzyl-amid)

(2-Benzyl-3-tert.-butylsulfonyl-propionyl)-Gly-AHCP-Ile-(N-3-guanidinomethyl-benzyl-amid)

(2-Benzyl-3-tert.-butylsulfonyl-propionyl)-Gly-AHCP-Leu-(N-3-guanidinomethyl-benzyl-amid)

(2-Benzyl-3-tert.-butylsulfonyl-propionyl)-ßAla-AHCP-Leu-AMPA

(2-Benzyl-3-tert.-butylsulfonyl-propionyl)-ßAla-AHCP-Ile-(N-2-amino-5,6-dimethyl-3-pyrazinyl-methyl-amid)

(2-Benzyl-3-tert.-butylsulfonyl-propionyl)-ßAla-AHCP-Leu-(N-2-amino-5,6-dimethyl-3-pyrazinyl-methyl-amid)

(2-Benzyl-3-tert.-butylsulfonyl-propionyl)-ßAla-AHCP-Ile-(N-3-aminomethyl-benzyl-amid)

(2-Benzyl-3-tert.-butylsulfonyl-propionyl)-ßAla-AHCP-Leu-(N-3-aminomethyl-benzyl-amid)

(2-Benzyl-3-tert.-butylsulfonyl-propionyl)-ßAla-AHCP-Ile-(N-3-guanidinomethyl-benzyl-amid)

(2-Benzyl-3-tert.-butylsulfonyl-propionyl)-ßAla-AHCP-Leu-(N-3-guanidinomethyl-benzyl-amid)

[4-(2-Benzyl-3-tert.-butylsulfonyl-propionyl-amino)-butyryl]-AHCP-Ile-AMPA

[4-(2-Benzyl-3-tert.-butylsulfonyl-propionyl-amino)-3-thia-butyryl]-AHCP-Ile-AMPA

[3-(2-Benzyl-3-tert.-butylsulfonyl-propionyl-amino)-3-oxa-propionyl]-AHCP-Ile-AMPA

4-Hydroxy-5-benzyl-6-(1,1-dimethylpropyl)-sulfonyl-hexanoyl-AHCP-Ome.

Beispiel 5

Analog Beispiel 3 erhält man "C" durch Kondensation von (2-Benzyl-3-tert.-butylsulfonyl-propionyl)-Gly-
AHCP-OH mit H-Ile-AMPA; F. 139-140°.

Analog erhält man

(2-Benzyl-3-tert.-butylsulfonyl-propionyl)-Gly-AHCP-Ile-(N-3-pyridylmethyl-amid)

(2-Benzyl-3-tert.-butylsulfonyl-propionyl)-Gly-AHCP-Abu-(N-3-pyridylmethyl-amid)

(2-Benzyl-3-tert.-butylsulfonyl-propionyl)-Gly-AHCP-Cal-(N-3-pyridylmethyl-amid)

(2-Benzyl-3-tert.-butylsulfonyl-propionyl)-Gly-AHCP-Leu-(N-3-pyridylmethyl-amid)

(2-Benzyl-3-tert.-butylsulfonyl-propionyl)-Gly-AHCP-Met-(N-3-pyridylmethyl-amid)

(2-Benzyl-3-tert.-butylsulfonyl-propionyl)-Gly-AHCP-Nle-(N-3-pyridylmethyl-amid)

(2-Benzyl-3-tert.-butylsulfonyl-propionyl)-Gly-AHCP-Nva-(N-3-pyridylmethyl-amid)

(2-Benzyl-3-tert.-butylsulfonyl-propionyl)-Gly-AHCP-Val-(N-3-pyridylmethyl-amid)

(2-Benzyl-3-tert.-butylsulfonyl-propionyl)-Gly-Sta-Ile-AMPA

(2-Benzyl-3-tert.-butylsulfonyl-propionyl)-Gly-Sta-Leu-AMPA

(2-Benzyl-3-tert.-butylsulfonyl-propionyl)-ßAla-Sta-Ile-AMPA

(2-Benzyl-3-tert.-butylsulfonyl-propionyl)-ßAla-Sta-Leu-AMPA

(2-Benzyl-3-tert.-butylsulfonyl-propionyl)-Gly-DACP-Ile-AMPA

(2-Benzyl-3-tert.-butylsulfonyl-propionyl)-Gly-DACP-Leu-AMPA

(2-Benzyl-3-tert.-butylsulfonyl-propionyl)-ßAla-DACP-Ile-AMPA

(2-Benzyl-3-tert.-butylsulfonyl-propionyl)-ßAla-DACP-Leu-AMPA

(2-Benzyl-3-tert.-butylsulfonyl-propionyl)-Gly-DACH-Ile-AMPA

(2-Benzyl-3-tert.-butylsulfonyl-propionyl)-Gly-DACH-Leu-AMPA

(2-Benzyl-3-tert.-butylsulfonyl-propionyl)-ßAla-DACH-Ile-AMPA

(2-Benzyl-3-tert.-butylsulfonyl-propionyl)-ßAla-DACH-Leu-AMPA

2-Isopropyl-4S-hydroxy-5S-[(2-benzyl-3-tert.-butylsulfonyl-propionyl)-Gly-amino]-7-methyl-octanoyl-Ile-AMPA
2-Isopropyl-4S-hydroxy-5S-[(2-benzyl-3-tert.-butylsulfonyl-propionyl)-Gly-amino]-7-methyl-octanoyl-Leu-AMPA
2-Isopropyl-4S-hydroxy-5S-[(2-benzyl-3-tert.-butylsulfonyl-propionyl)-ßAla-amino]-7-methyl-octanoyl-Ile-AMPA, F.218-219° ; Hydrochlorid, F. 185°
2-Isopropyl-4S-hydroxy-5S-[(2-benzyl-3-tert.-butylsulfonyl-propionyl)-ßAla-amino]-7-methyl-octanoyl-Leu-AMPA.

Beispiel 6

Analog Beispiel 3 erhält man aus 2-Benzyl-3-tert.-butylsulfonylpropionyl-Gly-AHCP-Ile-OH und H-Ala-AMPA das (2-Benzyl-3-tert.-butylsulfonylpropionyl)-Gly-AHCP-Ile-Ala-AMPA.

Beispiel 7

Analog Beispiel 3 erhält man "C" aus 2-Benzyl-3-tert.-butylsulfonylpropionyl-Gly-AHCP-Ile-OH und 5-Aminomethyl-4-amino-2-methyl-pyrimidin; F. 139-140° .
Analog erhält man
(2-Benzyl-3-tert.-butylsulfonyl-propionyl)-ßAla-AHCP-Ile-(N-2-pyridylmethyl-amid)
(2-Benzyl-3-tert.-butylsulfonyl-propionyl)-ßAla-AHCP-Ile-(N-3-pyridylmethyl-amid)
(2-Benzyl-3-tert.-butylsulfonyl-propionyl)-ßAla-AHCP-Ile-(N-2-hydroxy-4,6-dimethyl-3-pyridylmethyl-amid)
(2-Benzyl-3-tert.-butylsulfonyl-propionyl)-ßAla-AHCP-Ile-(N-4-aminomethyl-benzyl-amid)
(2-Benzyl-3-tert.-butylsulfonyl-propionyl)-ßAla-AHCP-Ile-(N-4-guanidinomethyl-benzyl-amid)
(2-Benzyl-3-tert.-butylsulfonyl-propionyl)-ßAla-AHCP-Ile-(N-4-dimethylaminomethyl-benzyl-amid)
(2-Benzyl-3-tert.-butylsulfonyl-propionyl)-ßAla-AHCP-Ile-(4-sulfamoyl-anilid)
(2-Benzyl-3-tert.-butylsulfonyl-propionyl)-ßAla-AHCP-Ile-(N-2-dimethylaminoethyl-amid) und dessen Methochlorid
(2-Benzyl-3-tert.-butylsulfonyl-propionyl)-ßAla-AHCP-Ile-(N-2-guanidinoethyl-amid)
(2-Benzyl-3-tert.-butylsulfonyl-propionyl)-ßAla-AHCP-Ile-[N-2-(N´-methylureido)-ethyl-amid]
(2-Benzyl-3-tert.-butylsulfonyl-propionyl)-ßAla-AHCP-Ile-[N-2-(N´-methyl-thioureido)-ethyl-amid]
(2-Benzyl-3-tert.-butylsulfonyl-propionyl)-ßAla-AHCP-Ile-(N-2-sulfoethyl-amid)
(2-Benzyl-3-tert.-butylsulfonyl-propionyl)-ßAla-AHCP-Ile-(N-2-sulfamoylethyl-amid)
(2-Benzyl-3-tert.-butylsulfonyl-propionyl)-ßAla-AHCP-Ile-[N-2-(N-methylsulfamoyl)-ethyl-amid]
(2-Benzyl-3-tert.-butylsulfonyl-propionyl)-ßAla-AHCP-Ile-[N-2-(N,N-diethylsulfamoyl)-ethyl-amid].

Beispiel 8

a) Analog Beispiel 3 erhält man aus 2-Benzyl-3-tert.-butylsulfonyl-propionyl-Gly-OH und 3-Oxo-4S-amino-5-cyclohexyl-pentanoyl-Ile-AMPA das 3-Oxo-4S-(2-benzyl-3-tert.-butylsulfonyl-propionyl)-Gly-amino-5-cyclohexyl-pentanoyl-Ile-AMPA.
b) Eine Lösung von 1 g des vorstehenden Keto-amids in 25 ml $CH_3OH$ wird an 0,1 g 10%ig. Pd-C bei 20° und 1 bar bis zum Stillstand der $H_2$-Aufnahme hydriert. Nach Filtrieren und Eindampfen erhält man ein Gemisch von 3R- und 3S-Hydroxy-4S-(2-benzyl-3-tert.-butylsulfonyl-propionyl)-Gly-amino-5-cyclohexyl-pentanoyl-Ile-AMPA.

Beispiel 9

Eine Lösung von 769 mg 3-Oxo-4S-(2-benzyl-3-tert.-butylsulfonyl-propionyl-Gly-amino)-5-cyclohexylpentanoyl-Ile-AMPA und 1,43 g $Na_2CO_3$ . 10 $H_2O$ in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert.
Man filtriert den Katalysator ab, dampft das Filtrat ein, trennt das erhaltene Gemisch an Kieselgel und erhält 3S-Amino-4S-(2-benzyl-3-tert.-butylsulfonyl-propionyl-Gly-amino)-5-cyclohexylpentanoyl-Ile-AMPA ("2-Benzyl-3-tert.-butylsulfonylpropionyl-Gly-DACP-Ile-AMPA"); daneben erhält man das 3R-Amino-Epimere.

Die nachfolgenden Beispiele betreffen pharmazeutische Zubereitungen.

Beispiel A: Tabletten

Ein Gemisch von 1 kg des Methansulfonats von 2-Benzyl-3-tert.-butylsulfonyl-propionyl)-ßAla-AHCP-Ile-AMPA, 4 kg Lactose, 1,2 kg Maisstärke, 200 g Talk und 100 g Magnesiumstearat wird in üblicher Weise zu Tabletten verpreßt, derart daß jede Tablette 200 mg Wirkstoff enthält.

Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

Beispiel C: Kapseln

500 g 2-Isopropyl-4S-hydroxy-5S-[(2-benzyl-3-tert.-butylsulfonyl-propionyl)-Gly-amino]-6-cyclohexyl-hexansäure-N-butylamid ("Isomer A") werden in üblicher Weise in Hartgelatinekapseln gefüllt, so daß jede Kapsel 500 mg Wirkstoff enthält.

Beispiel D: Injektionsgläser

Eine Lösung von 100 g "C"-Hydrochlorid in 4 l zweifach destilliertem Wasser wird mit 2n Salzsäure auf pH 6,5 eingestellt, steril filtriert und in Injektionsgläser abgefüllt. Man lyophilisiert unter sterilen Bedingungen und verschließt steril. Jedes Injektionsglas enthält 100 mg Wirkstoff.

Beispiel E: Suppositorien

Man schmilzt ein Gemisch von 50 g 2-Isopropyl-4S-hydroxy-5S-[(2-benzyl-3-tert.-butylsulfonyl-propionyl)-Gly-amino]-6-cyclohexyl-hexansäure-N-butyl-amid ("Isomer B") mit 10 g Sojalecithin und 140 g Kakaobutter, gießt in Formen und läßt erkalten. Jedes Suppositorium enthält 250 mg Wirkstoff.

**Ansprüche**

1. Aminosäurederivate der Formel I

$R^1$-Z-$(CH_2)_m$-CO-NR$^2$-CHR$^3$-CR$^4$-(CHR$^5$)$_n$-CO-E-Q-Y     I

worin

$R^1$     ($R^6$-$C_kH_{2k}$-S(O)$_x$-$C_rH_{2r}$)-L($R^7$-$C_pH_{2p}$)-CO- oder ($R^6$-$C_kH_{2k}$-S(O)$_x$-$C_rH_{2r}$)-L($R^7$-$C_pH_{2p}$)-CHOH-,

Z     -O-, -CH$_2$-, -CH=CH-, NR$^8$-, -NR$^8$-O-, -CH$_2$-O-, -CH$_2$-NR$^8$- oder -CH$_2$-S-,

E     0 bis 2 peptidartig miteinander verbundene Aminosäurereste ausgewählt aus der Gruppe bestehend aus Abu, Ala, Cal, His, Ile, Leu, Met, Nle, Nva, Phe, Trp, Tyr und Val,

Q     O oder NR$^9$,

Y     -$C_tH_{2t}$-$R^{1°}$, -$C_tH_{2t}$-$R^{11}$ oder -$C_wH_{2w}$-(CR$^{12}$)$_s$-$C_tH_{2t}$-$R^{1°}$,

$R^2$, $R^5$, $R^8$ und $R^9$     jeweils H oder A,

$R^3$, $R^6$, $R^7$ und $R^{1°}$     jeweils H, A, Ar, Ar-Alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^4$ und $R^{12}$     jeweils (H, OH), (H, NH$_2$) oder =O,

$R^{11}$     -SO$_3$H, -SO$_2$NH$_2$, -SO$_2$NHA, -SO$_2$NA$_2$, -NH$_2$, -NHA, -NA$_2$, -NA$_3^+$ An$^-$, -NH-C(=NH)-NH$_2$, -NH-C-(=NH)-NHCN, -NH-CO-NH$_2$, -NH-CO-NHA, -NH-CO-NA$_2$, -NH-CS-NH$_2$, -NH-CS-NHA oder -NH-CS-NA$_2$,

L     CH oder N,

14

k, p, r und t    jeweils 0, 1, 2, 3, 4 oder 5,

m    1, 2 oder 3,

n und s    jeweils 1 oder 2,

w    1, 2, 3, 4, 5, 6, 7, 8, 9 oder 10,

x    0, 1 oder 2,

Ar    unsubstituiertes oder ein- oder mehrfach durch A, OA, Hal, $CF_3$, OH, $NO_2$, Hydroxyalkyl, $NH_2$, NHA, $NA_2$, NHAc, SA, SO-A, $SO_2$-A, $SO_2NH_2$, $SO_2NHA$, CN, COOH, COOA, $CONH_2$, Aminoalkyl, HAN-alkyl, $A_2$N-Alkyl, $A_3N^+$-alkyl $An^-$ und/oder Guanidinyl-alkyl substituiertes Phenyl oder unsubstituiertes Naphthyl,

Het    einen gesättigten oder ungesättigten 5- oder 6-gliedrigen heterocyclischen Rest mit 1-4 N-, O-und/oder S-Atomen, der mit einem Benzolring kondensiert und/oder ein- oder mehrfach durch A, OA, Hal, $CF_3$, OH, $NO_2$, Carbonylsauerstoff, $NH_2$, NHA, $NA_2$, NHAc, SA, SO-A, $SO_2$-A, COOH, COOA, $CONH_2$, CN, $SO_2NH_2$, $NH-SO_2$-A, Ar, Ar-alkyl, Ar-alkenyl, Hydroxyalkyl und/oder Aminoalkyl substituiert sein kann und/oder dessen N- und/oder S-Heteroatome auch oxydiert sein können,

Hal    F, Cl Br oder J,

Ac    A-CO-, Ar-CO- oder A-NH-CO-,

$An^-$    ein Anion,

A    Alkyl mit 1-8 C-Atomen und

-alkyl-    eine Alkylengruppe mit 1-8 C-Atomen bedeuten,

worin ferner an Stelle einer oder mehrerer -NH-CO-Gruppen auch eine oder mehrere -NA-CO-Gruppen stehen können,

sowie deren Salze.

2.    a)    2-Isopropyl-4-hydroxy-5-[(2-benzyl-3-tert.-butylsulfonyl-propionyl)-Gly-amino]-6-cyclohexyl-hexansäure-N-butyl-amid;

b) (2-Benzyl-3-tert.-butylsulfonyl-propionyl)-ßAla-AHCP-Ile-(N-4-amino-2-methyl-5-pyrimidinyl-methyl-amid);

c) (2-Benzyl-3-tert.-butylsulfonyl-propionyl)-Gly-AHCP-Ile-(N-4-amino-2-methyl-5-pyrimidinylmethyl-amid).

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

$R^1$-$G^1$-OH

worin $G^1$

(a) fehlt und gleichzeitig $R^1$ ($R^6$-$C_kH_{2k}$-S(O)$_x$-$C_rH_{2r}$)-L($R^7$-$C_pH_{2p}$)-CO-bedeutet,

(b) -Z-$(CH_2)_m$-CO-,

(c) -Z-$(CH_2)_m$-CO-W-,

(d) -Z-$(CH_2)_m$-CO-W-$E^1$-,

(e) -Z-$(CH_2)_m$-CO-W-E- und

W    -$NR^2$-$CHR^3$-$CR^4$-$(CHR^5)_n$-CO- bedeuten

oder eines ihrer reaktionsfähigen Derivate

mit einer Verbindung der Formel III

H-$G^2$    III

worin $G^2$

(a) -$Z^1$-$(CH_2)_m$-CO-W-E-Q-Y

(b) -W-E-Q-Y,

(c) -E-Q-Y,

(d) -$E^2$-Q-Y,

(e) -Q-Y,

$Z^1$    -O-, -$NR^8$- oder -$NR^8$-O- und

$E^1$ + $E^2$ zusammen E bedeuten

oder einem ihrer reaktionsfähigen Derivate umsetzt,

und/oder daß man gegebenenfalls in einer Verbindung der Formel I eine funktionell abgewandelte Amino-und/oder Hydroxygruppe durch Behandeln mit solvolysierenden oder hydrogenolysierenden Mitteln in Freiheit setzt und/oder eine freie Amino- oder Hydroxygruppe acyliert und/oder eine Ketogruppe zu einer CHOH-Gruppe reduziert oder zu einer CH($NH_2$)-Gruppe reduktiv aminiert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen bei der Bekämpfung der reninabhängigen Hypertension oder des Hyperaldosteronismus.

7. Verwendung von Verbindungen der Formel I oder von deren physiologisch unbedenklichen Salzen zur Herstellung eines Arzneimittels zur Bekämpfung der reninabhängigen Hypertension oder des Hyperaldosteronismus.